# EUROPEAN PATENT SPECIFICATION

(11) **EP 4 447 891 B1**
(45) Date of publication and mention of the grant of the patent: **01.10.2025**
(21) Application number: 22840075.0
(22) Date of filing: 16.12.2022
(51) Int. Cl.: A61J 1/06

(54) **CONTAINER COMPRISING A LISDEXAMFETAMINE CONTAINING ORAL SOLUTION**
BEHÄLTER MIT EINER LISDEXAMFETAMINHALTIGEN ORALEN LÖSUNG
RÉCIPIENT COMPRENANT UNE LISDEXAMFÉTAMINE CONTENANT UNE SOLUTION ORALE

(30) Priority: 16.12.2021 EP 21386078
(43) Date of publication of application: 23.10.2024
(73) Proprietor: LABOMED PHARMACEUTICAL COMPANY S.A., 19441 Koropi, Attica (GR); Adalvo Limited, San Gwann, SGN 3000 (MT)
(72) Inventor: LIOLIOS, Georgios, 15232 Chalandri Attica (GR); PSARRAKIS, Ioannis, 19500 Lavrion (GR)
(74) Representative: Roukounas, Dimitrios
(86) International application number: PCT/EP2022/086486
(87) International publication number: WO 2023/111325

(56) References cited:
- WO-A1-2020/132310
- JP-B2- 4 064 261

## Description

### FIELD OF THE INVENTION

The present invention relates to a container comprising an oral solution of a pharmaceutically acceptable salt of lisdexamfetamine. Such a container is disclosed in the WO2020132310.

### BACKGROUND OF THE INVENTION

Lisdexamfetamine or L-lysine-d-amfetamine (LDX), (2S)-2,6-diamino-N-[(1S)-1-methyl-2-phenylethyl] hexanamide dimethanesulfonate) was first disclosed in US7105486.

Lisdexamfetamine has the following structure:

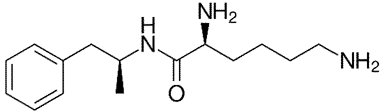

Medications comprising lisdexamfetamine, sold under the brand names *Elvanse*^{®} (DK, GB, SE), *Tyvense*^{®} (IE), *Venvanse*^{®} (BR), *Vyvanse*^{®} (US, CA) among others, are used to treat attention deficit hyperactivity disorder (ADHD) in people over the age of five as well as for moderate to severe binge eating disorder in adults.

Lisdexamfetamine is a pro-drug of dexamfetamine and contains D-amfetamine covalently linked to the essential amino acid L-lysine. It lacks stimulant properties but is hydrolyzed in the gut wall to release d-amfetamine. As it is an inactive material, it cannot be taken nasally, intravenously or in any other way to achieve an illicit stimulant effect. Furthermore, the metabolic hydrolysis of the pro-drug takes some time and as such the formulation has an element of in-built controlled release. The product can deliver dexamfetamine over a period of about 8 hours and so it is useful to treat ADHD in paediatric populations (aged 6 to 12), but the extent of its duration is not considered to be sufficient to treat adolescent and adult populations having much longer active days.

Lisdexamfetamine is currently commercially available only as hard capsules or chewable tablets. Each capsule or chewable tablet contains 10, 20, 30, 40, 50, 60 or 70 mg lisdexamfetamine dimesylate, equivalent to 5.8 mg, 11.6 mg, 17.3 mg, 23.1 mg, 28.9 mg, 34.7 mg or 40.3 mg of lisdexamfetamine, respectively).

Lisdexamfetamine dimesylate has the following structure:

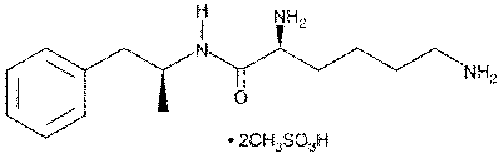

Although oral solid dosage forms such as capsules and chewable tablets are very popular, mainly because of ease of management, for certain users (e.g. children) these forms are not necessarily a convenient option, especially due to difficulty in swallowing these forms. This lack of convenience results in high incidence of non-compliance and ineffective therapy.

Although lisdexamfetamine acid addition salts, such as lisdexamfetamine dimesylate and lisdexamfetamine hydrochloride are very soluble in water, the desire for the development of an oral solution dosage form of a lisdexamfetamine salt is complicated by the fact that the molecule degrades significantly, mainly, at basic (alkaline), oxidative or other not easily defined degradation conditions.

According to WO 2006/121552, lisdexamfetamine can be formulated as an aqueous solution, or a sterile composition. The composition may be stored in freeze-dried form and may be associated with a stabilizing agent such as a carbohydrate. In use, the composition may be deployed in an aqueous solution containing salts, e.g., NaCl, detergents such as sodium dodecyl sulfate (SDS), and other components. Furthermore, according to WO 2006/121552 lisdexamfetamine may be formulated as a liquid dispersion for oral administration, for example as a syrup, emulsion, or suspension.

Patent application WO 2021/136602 describes aqueous oral solutions comprising a lisdexamfetamine salt, a buffer and at least one or more cosolvents selected from glycols and polyols, wherein the pH of the solution is from 5.5 to 9.0. According to WO 2021/136602, the final solutions are preferably filled in light-protective containers, such as amber type III glass 50 ml or 100 ml bottles sealed with child resistant, tamper evident screw caps.

There is a continuous necessity for drug products to have extended shelf lives (e.g., longer than twelve months), which is mainly highlighted by the retail pharmacy practice of routinely returning drug products with remaining shelf lives of less than twelve months. Furthermore, the necessity to have extended shelf lives especially at normal conditions is dictated by the fact that refrigeration of the drug products increases considerably the cost of treatment, and thus, further reduces the availability of the products to those in need.

The present invention provides a pharmaceutical product comprising a pharmaceutically acceptable salt of lisdexamfetamine with extended shelf life.

### SUMMARY OF INVENTION

The present invention provides a sealed vial comprising an oral solution of a pharmaceutically acceptable salt of lisdexamfetamine, wherein the vial is made of a plastic material consisting essentially of a synthetic organic polymer.

With the present invention the stability of the pharmaceutically acceptable salt of lisdexamfetamine in the oral solution is better than when the same oral solution is packaged in a vial made of glass. Thus, with the present invention the oral solution exhibits a longer shelf-life and can be stored at higher temperatures than when the same oral solution is packaged in a vial made of glass.

### DETAILED DESCRIPTION OF THE INVENTION

The present invention provides a sealed vial comprising an oral solution of a pharmaceutically acceptable salt of lisdexamfetamine, wherein the vial is made of plastic material, where the plastic material consists essentially of a synthetic organic polymer.

A vial (also known as a phial or flacon) is a small glass or plastic vessel or bottle, often used to store medication in the forms of liquids, powders or capsules. There are different types of vials such as single dose vials and multi-dose vials, which are often used for storing medications. A single dose vial is only used once whereas a multi-dose vial can be used more than once.

The term "sealed vial" as used herein, refers to a vial that is sealed by a cap (or closure). The cap maybe any article for closing a suitably shaped opening. It encompasses, but is not limited to, childproof closures, waterproof closures, pipette-associated caps, solid caps, plastic or polymeric caps.

Glass vials are often used in pharmaceuticals because they offer some important benefits that other materials do not offer. They are easy to sterilize with heat; colored glass has the ability to protect its content from certain electromagnetic wavelengths, such as the ultraviolet rays of the sun; they are chemically inert and will not react with their content; they are impermeable to water and air. More specifically, type III glass vials are the most common type of glass bottles that is used for packaging pharmaceutical non-parenteral preparations such as pharmaceutical oral solutions.

The present inventors have surprisingly found that, physicochemical stability of an oral solution comprising a pharmaceutically acceptable salt of lisdexamfetamine is enhanced when the solution is packaged/stored in a sealed vial made of plastic material, where the plastic material essentially consists of a synthetic organic polymer, instead of glass.

Furthermore, they have been found that the stability of a solution comprising a pharmaceutically acceptable salt of lisdexamfetamine is increased at even acidic and/or in strong alkaline pH regions when the solutions are packaged/stored in a sealed vial made of plastic material consisting essentially of a synthetic organic polymer, instead of glass.

Thus, the present invention provides a lisdexamfetamine-containing oral solution packaged in a sealed vial made of plastic material, where the plastic material consists essentially of a synthetic organic polymer.

The lisdexamfetamine-containing oral solution according to the invention exhibits excellent physicochemical stability at normal conditions of temperature (25°C) and relative humidity (60%), when packaged in a sealed vial consisting essentially of a synthetic organic polymer.

The present invention also provides a sealed vial comprising an oral solution of a pharmaceutically acceptable salt of lisdexamfetamine, wherein the vial consists essentially of a synthetic organic polymer.

The present invention further provides a method of increasing the physicochemical stability of an oral solution comprising a pharmaceutically acceptable salt of lisdexamfetamine, wherein the method includes the step of packaging the pharmaceutical oral solution in a sealed vial consisting essentially of a synthetic organic polymer.

The present invention further provides the use of a sealed vial made of a plastic material consisting essentially of a synthetic organic polymer for increasing the stability of an oral solution comprising a pharmaceutically acceptable salt of lisdexamfetamine.

The synthetic organic polymer according to the present invention is preferably selected from the group consisting of Polyethylene terephthalate (PET), High Density Polyethylene (HDPE), Low Density Polyethylene (LDPE), Linear Low Density Polyethylene (LLDPE), Polypropylene (PP), Polypropylene copolymer (PPCO), Polycarbonate (PC), Polyvinyl chloride (PVC), Polystyrene (PS), Phenol-formaldehyde (PF) and Fluoropolymer (FEP). More preferably, the synthetic organic polymer according to the present invention is selected from the group consisting of PET, HDPE, LDPE, LLDPE, PP and PPCO. Even more preferably, the synthetic organic polymer according to the present invention is selected from the group consisting of PET, LDPE, HDPE and PPCO. Most preferably, the synthetic organic polymer according to the present invention is PET.

Polyethylene terephthalate (PET or PETE) is a general-purpose thermoplastic polymer, which belongs to the polyester family of polymers. Polyester resins are known for their excellent combination of properties such as mechanical, thermal, chemical resistance as well as dimensional stability. PET is highly flexible, colorless and semi-crystalline resin in its natural state. Depending upon how it is processed, it can be semi-rigid to rigid. It shows good dimensional stability, resistance to impact, moisture, alcohols and solvents.

Commercially available PET grades include un-reinforced to glass reinforced, flame retardant and high flow materials for various engineering applications that typically require higher strength and or higher heat resistance. Addition of fillers like glass fibers, CNTs etc. help improve impact strength, surface finish, reduce warpage and several other benefits.

PET is available as a homopolymer and it can also be modified to produce copolymers (known as PETG or PET-G - polyethylene terephthalate glycol-modified) making it more desirable for a particular application. The common modifiers, which replace ethylene glycol or terephthalic acid to produce PETG are cyclohexane dimethanol (CHDM) and isophthalic acid respectively. There modifiers interfere with crystallization and lowers the polymer's melting temperature.

PET is also available as transparent or opaque PET. PET is made opaque by the addition of certain pigments.

Polyethylene (PE) or polythene is the most common polymer in use today. It is primarily used for packaging (e.g. in containers, including vials). Many kinds of PE are known, with most having the chemical formula (C₂H4)ₙ. PE is usually a mixture of similar polymers of ethylene, with various values of n. It can be low-density or high-density: low-density PE is extruded using high pressure and high temperature, while high-density PE is extruded using low pressure and low temperature. PE is usually thermoplastic, but it can be modified to become thermosetting instead, for example, in cross-linked PE.

Low Density Polyethylene (LDPE) is defined by a density that can range from 0.91 to 0.93 g/cm³. It withstands room temperature, although it can be reactive to oxidizing agents or some solvents, while it is flexible and tough.

High Density Polyethylene (HDPE) is defined by a density that can range from 0.94 to 0.97 g/cm³.

Linear Low Density Polyethylene (LLDPE) is a flexible, thermoplastic polymer. It is known for higher tensile strength and great resistance than LDPE.

Polypropylene (PP) also known as polypropene, is a thermoplastic polymer used in a wide variety of applications. It is produced via chain-growth polymerization from the monomer propylene. It has a density between 0.89 to 0.92 g/cm³ and it is the most durable polymer with higher thermal resistance abilities.

Polyvinyl chloride (PVC) is the world's third-most widely produced synthetic organic polymer (after PE and PP). PVC comes in two basic forms: rigid (sometimes abbreviated as RPVC) and flexible. The rigid form of PVC is usually used in making bottles. It can be made softer and more flexible by the addition of plasticizers, the most widely used being phthalates.

Polystyrene (PS) is a synthetic aromatic hydrocarbon polymer made from the monomer known as styrene. PS can be solid or foamed. General-purpose PS is clear, hard, and brittle and it is a poor barrier to oxygen and water vapour and has a relatively low melting point.

Phenol-formaldehyde (PF) or phenolic resins (also infrequently called phenoplasts) are synthetic polymers obtained by the reaction of phenol or substituted phenol with formaldehyde.

Fluoropolymer (FEP) is a fluorocarbon-based polymer with multiple carbon-fluorine bonds. It is characterized by a high resistance to solvents, acids, and bases. The best known fluoropolymer is polytetrafluoroethylene.

The vial, according to the invention, is sealed by a cap (or closure). The cap can be any article for closing a suitably shaped opening. It encompasses, but is not limited to, childproof closures, waterproof closures, pipette-associated caps, solid caps, plastic or polymeric caps. Preferably, the cap is screwed on the vial top or interlocked with the top of the vial.

The vial according to the invention may be opaque, semi-transparent or transparent (clear). Preferably, the vial according to the invention is opaque or semi-transparent. Even more preferably, the vial according to the invention is opaque.

An opaque vial, according to the invention, includes but is not limited to a blue type plastic vial, white type plastic vial, black type plastic vial, green type plastic vial, purple type plastic vial, brown type plastic vial and amber type plastic vial. Preferably the opaque vial, according to the invention, is an amber or brown type plastic vial. More preferably, the opaque vial is an amber type plastic vial.

A semi-transparent vial is typically a natural type plastic vial or milky white translucent type plastic vial.

Preferably, the sealed vial is waterproof, and, if possible, air-tight. For that, a sealing element may be required for the tightness of the system bottle-cap or bottle-pipette-cap or bottle-pipette or pipette-cap. This element can be supplied on its own and further fit in the bottle-neck, or around the pipette, or in the cap, or it can be previously adapted to the bottle, the cap or the pipette.

Optionally, exclusion of oxygen during filling of the pharmaceutical oral solution according to the invention to ensure that the amount of impurities resulting from degradation caused by oxidation or catalyzed by oxygen is maintained at a low level, may be also adopted. This may be achieved by either minimizing the volume of the headspace when the bottle is sealed so that the amount of air that remains in the headspace after sealing is too small to cause significant degradation, or by exclusion of oxygen when the oral solution is bottled by filling the headspace, or purging the bottle, with an inert gas, such as nitrogen or argon.

The oral solution of a pharmaceutically acceptable salt of lisdexamfetamine, according to the invention may be supplied as monodose or multidose preparation. Preferably the oral solution according to the invention is supplied as a multidose preparation.

Each dose from a plastic multidose sealed vial, can be administered by means of a device suitable for measuring the prescribed volume. The device is typically a spoon or a cup for volumes of 5 ml or multiples thereof, or an oral syringe for other volumes.

The oral solution according to the invention may comprise any pharmaceutically acceptable salt of lisdexamfetamine such as acetate, adipate, alginate, aspartate, benzoate, besylate, bisulfate, calcium edetate, carbonate, citrate, edetate, glutamate, hydrobromide, hydrochloride, hydroiodide, lactate, laurate, malate, maleate, mesylate, oleate, oxalate, pamoate, phosphate, saccharate, salicylate, stearate, succinate, sulfate, tartrate and tosylate salts, and the like. Preferably, the oral solution according to the invention comprises lisdexamfetamine dimesylate or lisdexamfetamine hydrochloride. More preferably, the oral solution according to the invention comprises lisdexamfetamine dimesylate.

The oral solutions according to the invention may also optionally contain additional excipients commonly used in preparing oral liquid compositions, such as cosolvents, stabilizers (or surfactants), buffers, antimicrobial preservatives, viscosity-adjusting agents, sweeteners and flavouring agents.

Cosolvents include but are not limited to maltitol, glycerol, mannitol, sorbitol, xylitol, propylene glycol, polyethylene glycol or any other pharmaceutically acceptable polyalkylene glycol product such as those known in the art as the "PEG" series, or mixtures thereof.

Stabilizers (or surfactants) include but are not limited to diethylene glycol monoethyl ether, or macrogol (15)-hydroxystearate or mixtures thereof.

Buffering agents include but are not limited to ascorbic acid, acetic acid, tartaric acid, citric acid monohydrate, sodium citrate, potassium citrate, acetic acid, sodium acetate, sodium hydrogen phosphate, sodium dihydrogen phosphate, calcium hydrogen phosphate, calcium dihydrogen phosphate or mixtures thereof.

Antimicrobial preservatives include but are not limited to sodium benzoate, benzoic acid, boric acid, sorbic acid and their salts thereof, benzyl alcohol, parahydroxy benzoic acids and their alkyl esters, methyl, ethyl and propyl parahydroxy benzoates and their salts or mixtures thereof.

Sweeteners include but are not limited to sucralose, aspartame, acesulfame-K, thaumatin, mogroside, saccharin and salts thereof, sodium cyclamate, glucose, sucrose, lactose, fructose, erythritol, glycyrrhizin, monosodium glycyrrhizinate, monoamonium glycyrrhizinate, dextrose or mixtures thereof.

Flavouring agents include but are not limited to fruit flavours such as orange, banana, strawberry, cherry, wild cherry, lemon and the like and other flavourings, such as cardamom, anise, mint, menthol, vanillin, bubble gum or mixtures thereof.

Preferably, the oral solution comprising a pharmaceutically acceptable salt of lisdexamfetamine according to the invention is aqueous.

Preferably, the oral solution comprises from 4 mg/ml to 32 mg/ml of a pharmaceutically acceptable salt of lisdexamfetamine. More preferably, the oral solution comprises from 8 mg/ml to 16 mg/ml of a pharmaceutically acceptable salt of lisdexamfetamine. Even more preferably, the oral solution comprises from 8 mg/ml to 16 mg/ml of lisdexamfetamine dimesylate.

The term "mg/ml" as used throughout the present application refers to mg of an ingredient per 1 ml of the oral solution.

Preferably, the oral solution comprises a pharmaceutically acceptable salt of lisdexamfetamine, and a pharmaceutically acceptable aqueous carrier comprising a pharmaceutically acceptable buffer, wherein the pH of the solution is from 4.5 to 9.0. More preferably, the pH of the solution is from 4.5 to 7.5. Even more preferably, the pH of the solution is from 5.0 to 7.0.

According to a preferred embodiment, the oral solution according to the invention comprises a pharmaceutically acceptable salt of lisdexamfetamine, and a pharmaceutically acceptable aqueous carrier comprising a pharmaceutically acceptable buffer and a cosolvent selected from the group consisting of a glycol and a polyol, wherein the pH of the solution is from 4.5 to 9.0. More preferably, the pH of the solution is from 4.5 to 7.5. Even more preferably, the pH of the solution is from 5.0 to 7.0. Preferably, the cosolvent is selected from maltitol, glycerol, mannitol, sorbitol, xylitol, propylene glycol, polyethylene glycols having an average molecular weight from 200 to 800, or a mixture thereof. More preferably, the cosolvent is selected from maltitol or polyethylene glycol. Even more preferably, the cosolvent is maltitol. Preferably, the total concentration of the cosolvent is from 5 mg/ml to 300 mg/ml, more preferably, from 50 mg/ml to 250 mg/ml and even more preferably, from 100 mg/ml to 200 mg/ml

The present invention also provides a pharmaceutical package including a sealed vial consisting essentially of a synthetic organic polymer, wherein the vial comprises an oral solution comprising a pharmaceutically acceptable salt of lisdexamfetamine and a leaflet containing information and instructions to a patient for the use of the lisdexamfetamine oral solution.

### EXAMPLES

### EXAMPLE 1

The purpose of this experiment was to evaluate the influence of vials made of different materials and the pH of the solution in the stability of lisdexamfetamine dimesylate solutions.

The lisdexamfetamine dimesylate solutions were prepared in the following manner:
Purified water was added into a vessel. Lisdexamfetamine dimesylate and the cosolvent, if present, were dissolved into purified water under stirring. A buffer solution, prepared in a different vessel, was added under continuous stirring until lisdexamfetamine dimesylate was completely dissolved. Preservatives were also added under continuous stirring until complete dissolution. The flavour was then added under continuous stirring, until complete dissolution. The pH of the solution was adjusted with a quantity of the buffer solution to the desired value. Finally, the volume was adjusted with purified water. The final solution was filled in 50 mL amber type class III glass bottles or in 50 mL amber type plastic (PET, PPCO, HDPE) bottles or milky white translucent LDPE bottles. In both cases the bottles were sealed with child resistant, tamper evident screw caps.

Storage conditions of temperature (40°C) and relative humidity (75%) applied for a total period of six months. Quantification of lisdexamfetamine dimesylate and its impurities in the compositions was performed by HPLC.

**Table 1**

| | **Composition** | | | |
|---|---|---|---|---|
| | **A** | **B** | **C** | **D** |
| **Active ingredient** | mg/ml | | | |
| Lisdexamfetamine dimesylate | 12 | 12 | 12 | 12 |

| **Excipients** | | | | |
|---|---|---|---|---|
| Maltitol | - | 300 | 300 | 300 |
| Sodium methyl paraben | 0.2 | 0.2 | 0.2 | 0.2 |
| Sodium propyl paraben | 0.1 | 0.1 | 0.1 | 0.1 |
| Flavour mint | 0.5 | 0.5 | 0.5 | 0.5 |
| Purified water | q.s. 1ml | q.s. 1ml | q.s. 1ml | q.s. 1ml |
| Phosphate buffer solution | pH: 6.0 | pH: 6.0 | pH:4.5 | pH: 7.5 |

**Table 2: Initial impurity results, T=0**

| | **Compositions** | | | |
|---|---|---|---|---|
| | **A** | **B** | **C** | **D** |
| | **Impurity results** | | | |
| **Opaque amber, PET** | Dextroamfetamine: N.D. | | | |
| **Amber, glass type III** | Z-Lys-(Z)-Dextroamphetamine: N.D. | | | |
| **Milky white, LDPE** | D-Lisdexamphetamine: N.D. | | | |
| **Opaque amber, PPCO** | Max Unknown: N.D. | | | |
| **Opaque amber, HDPE** | Total impurities; N.D. | | | |

| | | | | |
|---|---|---|---|---|
| N.D.: Not Detected | | | | |

As seen by the impurity results presented in Table 2, none of the known, specified impurities of lisdexamfetamine i.e. dextroamphetamine, Z-Lys-(Z)-dextroamphetamine, D-lisdexamphetamine, were detected at T=0. Further to that, no other peak that can be attributed to any unknown, unspecified impurity of Lisdexamfetamine was observed in the chromatogram.

In the following table 3, "maximum unknown impurity" refers to an unknown (not specified) impurity of lisdexamfetamine, which in the chromatogram gives the greater peak and thus corresponds to the greater impurity concentration in the solution, while "total impurities" is the sum of all known (specified) and unknown (not specified) impurities of lisdexamfetamine observed in the chromatogram.

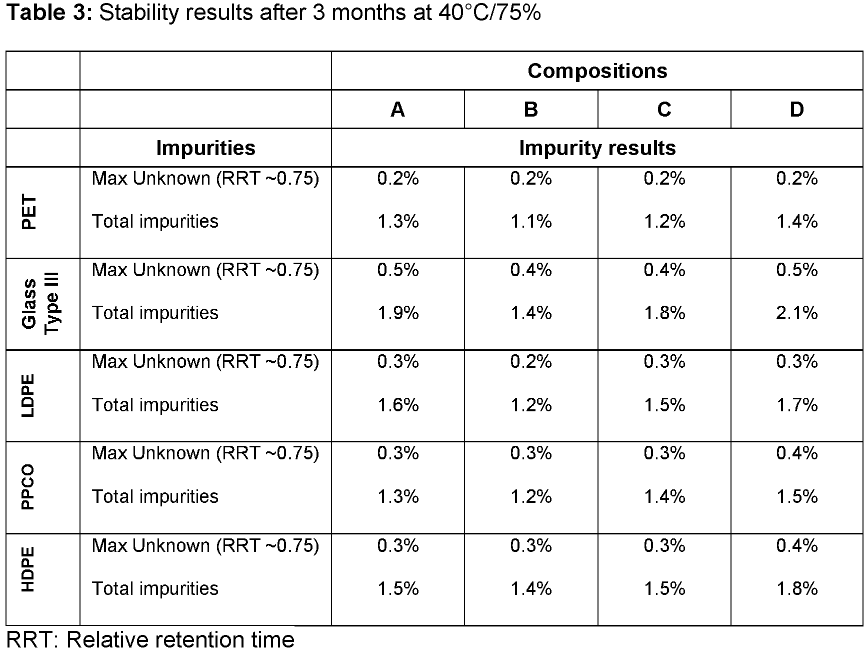

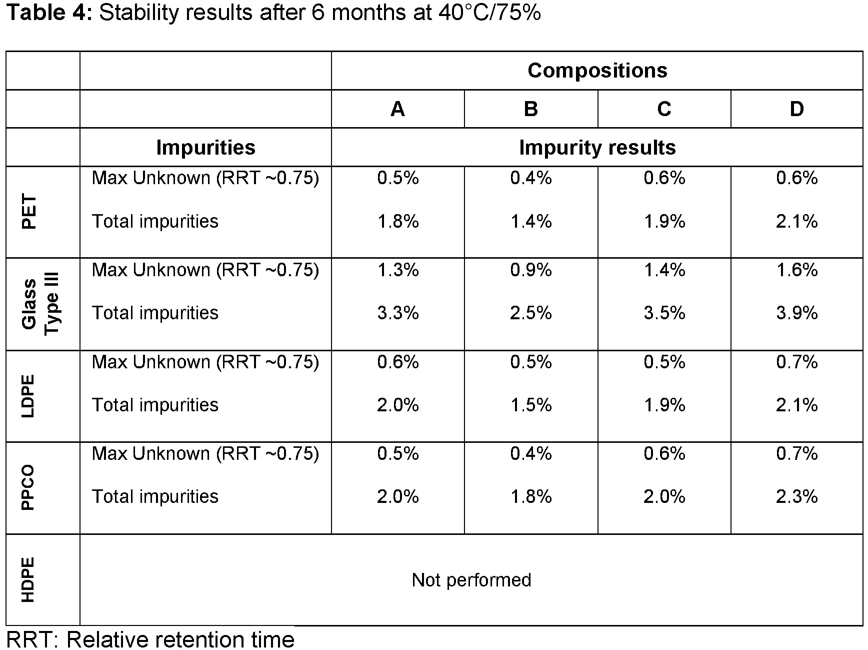

From this study, it is surprisingly concluded that the physicochemical stability of lisdexamfetamine dimesylate salt in an aqueous solution is surprisingly enhanced without the need of addition of a non-aqueous cosolvent (composition A) when the solution is packaged in a plastic bottle, instead of a glass bottle.

It has also been found that for solutions stored in plastic vials the control of pH is not critical for the stability of lisdexamfetamine dimesylate solutions with pH above 4.5.

Repetition of the above study with a composition such as composition B, in which the pH of the solution was about 3.5, gave a max unknown impurity at RRT ~0.75 of 1.6% and total impurities of 3.8%, when packaged in a sealed HDPE bottle for a time period of 6 months at 40°C/75% and a max unknown impurity at RRT ~0.75 of 1.9% and total impurities of 4.4%, when packaged in a sealed glass type III bottle.

### EXAMPLE 2

The purpose of this experiment was to evaluate the influence of vials in the stability of two lisdexamfetamine dimesylate solutions disclosed in WO 2021/136602 when the solutions are stored in glass type III sealed bottles or PET sealed bottles.

These lisdexamfetamine dimesylate solutions were prepared in the following manner:
Purified water was added into a vessel. Lisdexamfetamine dimesylate and the cosolvent, were successively dissolved into purified water under stirring. A buffer solution, prepared in a different vessel, was added under continuous stirring until lisdexamfetamine dimesylate was completely dissolved. Preservative was also added under continuous stirring until complete dissolution. The flavour was then added under continuous stirring, until complete dissolution. The pH of the solution was adjusted with a quantity of the buffer solution to the desired value. Finally, the volume was adjusted with purified water. A quantity of the final solution was filled in 50 mL amber type class III glass bottles and the remaining solution was filed in 50 mL amber type plastic-PET bottles. In both cases the bottles were sealed with child resistant, tamper evident screw caps.

Storage conditions of temperature (40°C) and relative humidity (75%) applied for a total period of six months and of temperature (25°C) and relative humidity (60%) applied for a total period of twelve months. Quantification of lisdexamfetamine dimesylate and its impurities, in the compositions was performed by HPLC.

**Table 5: Oral solutions according to WO 2021/136602**

| | **Composition IV** | **Composition III** |
|---|---|---|
| **Active ingredient** | mg/ml | |
| Lisdexamfetamine dimesylate | 10 | 10 |

| **Excipients** | | |
|---|---|---|
| Glycerol | 100 | - |
| PEG-400 | - | 100 |
| Sodium benzoate | 0.1 | 0.1 |
| Flavour mint | 0.5 | 0.5 |
| Purified water | q.s. 1ml | q.s. 1ml |
| Phosphate buffer solution | pH: 6.5 | pH: 6.5 |

**Table 6: Stability results, oral solutions according to WO 2021/136602**

| | **Parameter** | **Composition IV** | **Composition III** |
|---|---|---|---|
| T=0 | Assay | 100.3% / 100.9% | 99.9% / 100.5% |
| | Max Impurities | ND / ND | ND / ND |
| **Glass** /**PET** | Total impurities | ND / ND | ND / ND |
| 40°C/75%RH 4 weeks | Assay | 99.2% / 100.5% | 99.6% / 100.3% |
| | Max Impurities | RRT 0.70: 0.09% / 0.02% | D-Ldxamfet: 0.06% / 0.06% |
| | | RRT 0.72: 0.12% / 0.05% | |
| **Glass** / **PET** | Total impurities | 0.24% / 0.08% | 0.06% / 0.06% |
| 40°C/75%RH 6 months | Assay | 98.6% / 99.5% | 98.5% / 100.0% |
| | Impurities | RRT 0.50: 0.4% / 0.2% | RRT 0.50: 0.5% / 0.2% |
| | | RRT 0.72: 0.8% / 0.4% | RRT 0.72: 0.8% / 0.3% |
| **Glass** / **PET** | Total impurities | 2.6% / 1.5% | 2.4% / 1.4% |
| 25°C/60%RH 9 months | Assay | 99.7% / 100.2% | 99.4% / 99.8% |
| | Max Impurities | RRT 0.50: 0.1% / 0.1% | RRT 0.50: 0.1% / 0.1% |
| | | RRT 0.72: 0.2% / 0.1% | RRT 0.72: 0.2% / 0.1% |
| **Glass** / **PET** | Total impurities | 0.6% / 0.3% | 0.3% / 0.2% |
| 25°C/60%RH 12 months | Assay | 99.1% / 99.9% | 99.2% / 99.6% |
| | Max Impurities | RRT 0.50: 0.2% / 0.1% | RRT 0.50: 0.1% / 0.1% |
| | | RRT 0.72: 0.3% / 0.1% | RRT 0.72: 0.3% / 0.1% |
| **Glass** / **PET** | Total impurities | 0.8% / 0.3% | 0.4% / 0.3% |
| 4°C 12 months | Assay | 99.8% / 100.3% | 99.6% / 99.7% |
| | Max Impurities | RRT 0.72: 0.1% / 0.1% | RRT 0.72: 0.1% / 0.1% |
| **Glass** / **PET** | Total impurities | 0.3% / 0.3% | 0.3% / 0.2% |

| | | | |
|---|---|---|---|
| RRT: Relative retention time, D-Ldxamfet: D-lisdexamfetamine | | | |

Surprisingly it was found that two of the pharmaceutical oral solutions described in WO 2021/136602, when packaged/stored in a glass type III sealed bottle are less stable than when they are packaged/stored in a PET sealed bottle.

According to EU guidelines "ICH Q3A (R2) Impurities in new drug substances" the identification threshold i.e. the limit above (>) which a degradation product should be identified is 0.2% (at 25°C/60%) for an pharmaceutical oral solution comprising 10 mg/ml lisdexamfetamine dimesylate.

The above results show that refrigeration at e.g. 2° C to 8° C is needed for keeping the solutions described in WO 2021/136602 stable for more than twelve months, when they are packaged/stored in a glass type III bottle. However, refrigeration of drug products increases considerably the cost of treatment of a patient, and thus, further reduces its availability to those in need.

On the other hand, it has been found that, physicochemical stability of lisdexamfetamine dimesylate in an aqueous solution is surprisingly enhanced when the solution is packaged/stored in a PET sealed bottle, instead of a glass bottle. Importantly, the shelf-life of the solution filled in the PET bottles can be extended for at least 3 months (compared to the self-life when the solution is filled in a glass bottle) at normal conditions of temperature (25°C) and relative humidity (60%).

## Claims

1. A sealed vial comprising an oral solution of a pharmaceutically acceptable salt of lisdexamfetamine, wherein the vial is made of plastic material, where the plastic material consists essentially of a synthetic organic polymer.

2. The sealed vial according to claim 1, wherein the synthetic organic polymer is selected from the group consisting of polyethylene terephthalate, high density polyethylene, low density polyethylene, linear low density polyethylene, polypropylene, polypropylene copolymer, polycarbonate, polyvinyl chloride, polystyrene, phenol-formaldehyde and fluoropolymer.

3. The sealed vial according to claim 1 or 2, wherein the synthetic organic polymer is selected from the group consisting of polyethylene terephthalate, low density polyethylene, high density polyethylene, and polypropylene copolymer.

4. The sealed vial according to any one of the preceding claims, wherein the synthetic organic polymer is polyethylene terephthalate.

5. The sealed vial according to any one of the preceding claims, wherein the vial is opaque, semi-transparent or transparent.

6. The sealed vial according to any one of the preceding claims, wherein the vial is opaque.

7. The sealed vial according to any one of the preceding claims, wherein the vial is an amber type, polyethylene terephthalate vial.

8. The sealed vial according to any one of the preceding claims, wherein the oral solution is aqueous.

9. The sealed vial according to claim 8, wherein the pH of the oral solution is from 4.5 to 9.

10. The sealed vial according to claim 9, wherein the pH of the oral solution is from 5.0 to 7.0.

11. The sealed vial according to any one of claims 8 to 10, wherein the oral solution comprises a cosolvent selected from the group consisting of maltitol, glycerol, mannitol, sorbitol, xylitol, propylene glycol, polyethylene glycol and mixtures thereof.

12. The sealed vial according to claim 11, wherein the cosolvent is selected from maltitol or polyethylene glycol.

13. The sealed vial according to any one of the preceding claims, wherein the pharmaceutically acceptable salt of lisdexamfetamine is selected from the group consisting of acetate, adipate, alginate, aspartate, benzoate, besylate, bisulfate, calcium edetate, carbonate, citrate, edetate, glutamate, hydrobromide, hydrochloride, hydroiodide, lactate, laurate, malate, maleate, mesylate, oleate, oxalate, pamoate, phosphate, saccharate, salicylate, stearate, succinate, sulfate, tartrate and tosylate.

14. The sealed vial according to any one of the preceding claims, wherein the pharmaceutically acceptable salt of lisdexamfetamine is the dimesylate or the hydrochloride.

15. A pharmaceutical package comprising a sealed vial as defined in any one of the preceding claims and a leaflet containing information and instructions to a patient. for the use of the lisdexamfetamine oral solution.

## Patentansprüche

1. Versiegelte Durchstechflasche, umfassend eine orale Lösung eines pharmazeutisch verträglichen Salzes von Lisdexamfetamin, wobei die Durchstechflasche aus Kunststoffmaterial hergestellt ist, wobei das Kunststoffmaterial im Wesentlichen aus einem synthetischen organischen Polymer besteht.

2. Versiegelte Durchstechflasche nach Anspruch 1, wobei das synthetische organische Polymer aus der Gruppe ausgewählt ist, die aus Polyethylenterephthalat, Polyethylen hoher Dichte, Polyethylen niedriger Dichte, linearem Polyethylen niedriger Dichte, Polypropylen, Polypropylen-Copolymer, Polycarbonat, Polyvinylchlorid, Polystyrol, Phenol-Formaldehyd und Fluorpolymer besteht.

3. Versiegelte Durchstechflasche nach Anspruch 1 oder 2, wobei das synthetische organische Polymer aus der Gruppe ausgewählt ist, die aus Polyethylenterephthalat, Polyethylen niedriger Dichte, Polyethylen hoher Dichte und Polypropylen-Copolymer besteht.

4. Versiegelte Durchstechflasche nach einem der vorstehenden Ansprüche, wobei das synthetische organische Polymer Polyethylenterephthalat ist.

5. Versiegelte Durchstechflasche nach einem der vorstehenden Ansprüche, wobei die Durchstechflasche undurchsichtig, halbtransparent oder transparent ist.

6. Versiegelte Durchstechflasche nach einem der vorstehenden Ansprüche, wobei die Durchstechflasche undurchsichtig ist.

7. Versiegelte Durchstechflasche nach einem der vorstehenden Ansprüche, wobei die Durchstechflasche eine Durchstechflasche vom bernsteinfarbenen Typ aus Polyethylenterephthalat ist.

8. Versiegelte Durchstechflasche nach einem der vorstehenden Ansprüche, wobei die orale Lösung wässrig ist.

9. Versiegelte Durchstechflasche nach Anspruch 8, wobei der pH der oralen Lösung von 4,5 bis 9 beträgt.

10. Versiegelte Durchstechflasche nach Anspruch 9, wobei der pH der oralen Lösung von 5,0 bis 7,0 beträgt.

11. Versiegelte Durchstechflasche nach einem der Ansprüche 8 bis 10, wobei die orale Lösung ein Co-Lösungsmittel umfasst, das aus der Gruppe ausgewählt ist, die aus Maltit, Glycerin, Mannit, Sorbit, Xylit, Propylenglykol, Polyethylenglykol und Mischungen davon besteht.

12. Versiegelte Durchstechflasche nach Anspruch 11, wobei das Co-Lösungsmittel aus Maltit oder Polyethylenglykol ausgewählt ist.

13. Versiegelte Durchstechflasche nach einem der vorstehenden Ansprüche, wobei das pharmazeutisch verträgliche Salz von Lisdexamfetamin aus der Gruppe ausgewählt ist, die aus Acetat, Adipat, Alginat, Aspartat, Benzoat, Besylat, Bisulfat, Calciumedetat, Carbonat, Citrat, Edetat, Glutamat, Hydrobromid, Hydrochlorid, Hydroiodid, Lactat, Laurat, Malat, Maleat, Mesylat, Oleat, Oxalat, Pamoat, Phosphat, Saccharat, Salicylat, Stearat, Succinat, Sulfat, Tartrat und Tosylat ausgewählt ist.

14. Versiegelte Durchstechflasche nach einem der vorstehenden Ansprüche, wobei das pharmazeutisch verträgliche Salz von Lisdexamfetamin das Dimesylat oder das Hydrochlorid ist.

15. Pharmazeutische Verpackung, umfassend eine versiegelte Durchstechflasche nach einem der vorstehenden Ansprüche und eine Packungsbeilage mit Informationen und Anweisungen für einen Patienten zur Verwendung der oralen Lisdexamfetamin-Lösung.

## Revendications

1. Récipient scellé comprenant une solution orale d'un sel pharmaceutiquement acceptable de lisdexamfétamine, le récipient étant constitué d'un matériau en plastique, où le matériau en plastique est essentiellement constitué d'un polymère organique synthétique.

2. Récipient scellé selon la revendication 1, dans lequel le polymère organique synthétique est choisi dans le groupe constitué par le téréphtalate de polyéthylène, le polyéthylène haute densité, le polyéthylène basse densité, le polyéthylène basse densité linéaire, le polypropylène, un copolymère de polypropylène, le polycarbonate, le chlorure de polyvinyle, le polystyrène, le phénol-formaldéhyde et un fluoropolymère.

3. Récipient scellé selon la revendication 1 ou 2, dans lequel le polymère organique synthétique est choisi dans le groupe constitué par le téréphtalate de polyéthylène, le polyéthylène basse densité, le polyéthylène haute densité et un copolymère de polypropylène.

4. Récipient scellé selon l'une quelconque des revendications précédentes, dans lequel le polymère organique synthétique est le téréphtalate de polyéthylène.

5. Récipient scellé selon l'une quelconque des revendications précédentes, dans lequel le récipient est opaque, semi-transparent ou transparent.

6. Récipient scellé selon l'une quelconque des revendications précédentes, dans lequel le récipient est opaque.

7. Récipient scellé selon l'une quelconque des revendications précédentes, dans lequel le récipient est un récipient de téréphalate de polyéthylène, de type ambre.

8. Récipient scellé selon l'une quelconque des revendications précédentes, dans lequel la solution orale est aqueuse.

9. Récipient scellé selon la revendication 8, dans lequel le pH de la solution orale est de 4,5 à 9.

10. Récipient scellé selon la revendication 9, dans lequel le pH de la solution orale est de 5,0 à 7,0.

11. Récipient scellé selon l'une quelconque des revendications 8 à 10, dans lequel la solution orale comprend un cosolvant choisi dans le groupe constitué par le maltitol, le glycérol, le mannitol, le sorbitol, le xylitol, le propylène glycol, le polyéthylène glycol et leurs mélanges.

12. Récipient scellé selon la revendication 11, dans lequel le cosolvant est choisi parmi le maltitol ou le polyéthylène glycol.

13. Récipient scellé selon l'une quelconque des revendications précédentes, dans lequel le sel pharmaceutiquement acceptable de lisdexamfétamine est choisi dans le groupe constitué par l'acétate, l'adipate, l'alginate, l'aspartate, le benzoate, le bésylate, le bisulfate, l'édétate de calcium, le carbonate, le citrate, l'édétate, le glutamate, le bromhydrate, le chlorhydrate, l'iodohydrate, le lactate, le laurate, le malate, le maléate, le mésylate, l'oléate, l'oxalate, le pamoate, le phosphate, le saccharate, le salicylate, le stéarate, le succinate le sulfate, le tartrate et le tosylate.

14. Récipient scellé selon l'une quelconque des revendications précédentes, dans lequel le sel pharmaceutiquement acceptable de lisdexamfétamine est le dimésylate ou le chlorhydrate.

15. Conditionnement pharmaceutique comprenant un récipient scellé selon l'une quelconque des revendications précédentes et un mode d'emploi contenant les informations et les instructions à un patient, pour l'utilisation de la solution orale de lisdexamfétamine.
